# EUROPEAN PATENT SPECIFICATION

(11) **EP 4 262 590 B1**
(45) Date of publication and mention of the grant of the patent: **17.04.2024**
(21) Application number: 21835847.1
(22) Date of filing: 14.12.2021
(51) Int. Cl.: A61B 17/34, A61B 90/11

(54) **SUPPORT DEVICE TO GUIDE TREATMENT INSTRUMENTS IN CLINICAL PROCEDURES**
UNTERSTÜTZUNGSGERÄT ZUR FÜHRUNG VON BEHANDLUNGSINSTRUMENTEN BEI KLINISCHEN VERFAHREN
DISPOSITIF DE SOUTIEN POUR GUIDER LES INSTRUMENTS DE TRAITEMENT DANS LES PROCÉDURES CLINIQUES

(30) Priority: 16.12.2020 IT 202000030962
(43) Date of publication of application: 25.10.2023
(73) Proprietor: H.S. - Hospital Service S.P.A., 00178 Roma (RM) (IT)
(72) Inventor: AMABILE, Claudio, 04012 Cisterna di Latina LT (IT); IACOBBI, Massimiliano, 00040 Ardea RM (IT); TOSORATTI, Nevio, 00148 Roma RM (IT)
(74) Representative: Società Italiana Brevetti S.p.A.
(86) International application number: PCT/IB2021/061684
(87) International publication number: WO 2022/130193

(56) References cited:
- WO-A1-2019/180154
- WO-A2-2013/106569
- US-A- 5 693 032

## Description

### Technical field of the invention

The present invention relates to the field of the medical devices. In particular, it relates to a support element to guide the motion of treatment instruments inside a patient's body, incorporating an improved system for adjusting the position of the access point.

### Background

Many clinical treatments require the positioning in the patient's body of suitable medical instruments configured to supply the treatment at *target* tissues inside the body after said instruments, or at least a portion thereof, have crossed healthy tissues not intended to be treated. Such treatments include, by way of example and not for exhaustive purposes, biopsy, ablation, administration of substances and neurosurgical operations.

In order to maximize the treatment effect on the target tissues, by reducing to the minimum the unwished effects on the healthy tissues, an accurate positioning of said instruments is required.

It is known to use remote *imaging* techniques such as ultrasound scanning, computed tomography or magnetic resonance. The remote *imaging* allows to display the *target* and the treatment medical device even when they are incorporated in the tissues or otherwise hidden by visually opaque matter. This allows to plan the route for inserting the device before starting the procedure and to confirm the correct positioning thereof before supplying the treatment.

Alongside the remote *imaging* techniques, it is also known to provide mechanical support for the positioning of the treatment device. Solutions of this type are described for example in US5263956A, WO2004/021898, WO2004/093720 and WO2005/030075.

Said support devices typically comprise a base element to fasten the device itself to the patient's body, at least a stiff body operatively coupled to the base and provided with guiding means to constrain the insertion/extraction trajectory of the treatment device, and means for adjusting the relative angular position between the stiff body and the base element. In particular, among the known adjusting means it is possible to mention ball joints and rotatable half-circular guiding pairs.

The point in which the trajectory defined by the guiding means crosses the surface of the base element (which, in use, is in contact with the patient's body) allows to determine the entry point of the treatment device in the patient's body.

According to some types of known techniques, the typical steps for positioning a medical device providing a clinical treatment (hereinafter designated in short as "treatment device") at target tissues inside a patient's body provide for:
- obtaining a scanning of the target tissues and of the surrounding healthy tissues;
- detecting a wished insertion trajectory for the treatment medical device;
- determining the wished entry point for the treatment device on the surface of the patient's body;
- applying a support device to said surface so that the entry point of the treatment device determined with the support device coincides as much as possible with the wished entry point;
- further adjusting the angular position of the stiff body with the purpose of obtaining a position congruent to the wished trajectory;
- coupling the treatment medical device with the support device;
- positioning the treatment medical device in the patient's body at the target tissues.

However, the known techniques have some drawbacks.

For example, the devices based upon ball joints of the type described in US5263956A involve a shifting of the real entry point in case the entry trajectory of the treatment device is subsequently modified, due to the raising of the rotation centre of the ball joint with respect to the surface of the patient's body. This effect is limited in space, it depends upon the entry trajectory and it is clearly unwished, as moreover discussed in WO 2004/021898. An additional example of ball joint is described in WO2019/180154 A1 and it is used for stabilizing the treatment devices. A system for anchoring to a patient's body for catheters and/or lines for fluid supply or drainage is described in US5693032A.

An additional example is provided by WO2013/106569 A2 which discloses systems and methods related to surgical procedures in which the entry point in the patient's body, in particular a blown cavity, is predetermined and integral to the patient's body once the device is anchored thereto and in which said systems and methods allow to make access and to position surgical devices inside said cavity.

In general terms, the known support and guiding devices do not allow a free and substantial adjustment of the entry point of the treatment medical device inside the patient's body once it has been applied to the body surface.

### Brief description of the invention

The technical problem placed and solved by the present invention is then to overcome the above-illustrated problems and, in particular, to provide a support element to guide the insertion and/or the extraction of a treatment medical device in/from a patient's body at a target tissue, as defined in claim 1.

Further features of the present invention are defined in the corresponding depending claims.

The invention relates to a device or support element to guide treatment medical devices during clinical procedures towards a designated therapeutic target. The invention provides for means for the reversible anchoring of the support element to the patient's body and a main body provided with means for constraining the motion of the above-mentioned treatment devices.

The main body comprises a first and a second component which can be coupled to each other along a coupling direction, which direction is orthogonal to a contact surface of the first component with the patient's body.

Said components comprise corresponding through-openings configured to constrain the motion of the treatment device when crossing the support element and to determine at least one access point on the patient's body.

The device of the invention further comprises locking means of the first and of the second component, which locking means is movable between an operating condition and a positioning condition.

Under said operating condition, the first and the second components are relatively locked in such a way as to retain therebetween the anchoring means and, under said positioning condition, they are movable with respect to the anchoring means in an adjustment plane parallel to said contact surface to adjust the position of said at least one access point.

Advantageously, the invention then allows to adjust the insertion or extraction point of the treatment device in the patient's body when the device itself results to be associated (for example inserted) to the support element.

According to an additional advantageous aspect, the present invention allows to compensate unwished deviations of the position of the access point actually obtained during the operation with respect to the wished access point. Such deviations can be due, for example, to the operator's uncertainties, to the change in the position of the target tissue with respect to the body surface due to breathing or other motions of the internal organs, to deficiencies in the operation planning phase.

Still, the determination of the insertion/extraction direction (or trajectory), which typically is performed only based upon the patient's body image without a real presence of the support element, can be re-calibrated after having fastened the support element itself on the patient, by allowing to reduce the operation time and to improve the accuracy thereof. This is particularly relevant when it is necessary to use contemporarily on the target tissue a plurality of treatment medical devices through the support element.

Other advantages, together with the features and use modes of the present invention, will result evident from the following detailed description of preferred embodiments thereof, shown by way of example and not for limitative purposes.

### Brief description of figures

The drawings shown in the enclosed figures will be referred to, wherein:
- Figure 1 shows a schematic side view illustrating an operating configuration of a support device according to present invention;
- Figure 2 shows a perspective view exemplifying a preferred embodiment of the anchoring means of the support device according to the present invention;
- Figure 3A shows an overall view of the support device according to a first embodiment of the present invention;
- Figure 3B shows a cross-section view of the device of Figure 3A;
- Figures 3C and 3D show an exploded view of a component of the main body of the device of Figure 3A and an axonometric view of the mode for coupling the locking means with a component of the main body of the device of Figure 3A, respectively;
- Figure 4A shows an overall view of the support device according to a second embodiment of the present invention;
- Figure 4B and 4C show a front section view and a cross-section view of the device of Figure 4A, respectively;
- Figures 4D and 4E show an exploded view of a component of the main body of the device of Figure 4A and an axonometric view of the way for coupling the locking means with (a portion) of the main body of the device of Figure 4A, respectively.

The thicknesses and the bends shown in the above-mentioned Figures have to be meant as purely exemplifying and they are not necessarily shown in proportion.

### Detailed description of embodiments of the invention

The present invention will be described hereinafter by making reference to the above-mentioned Figures.

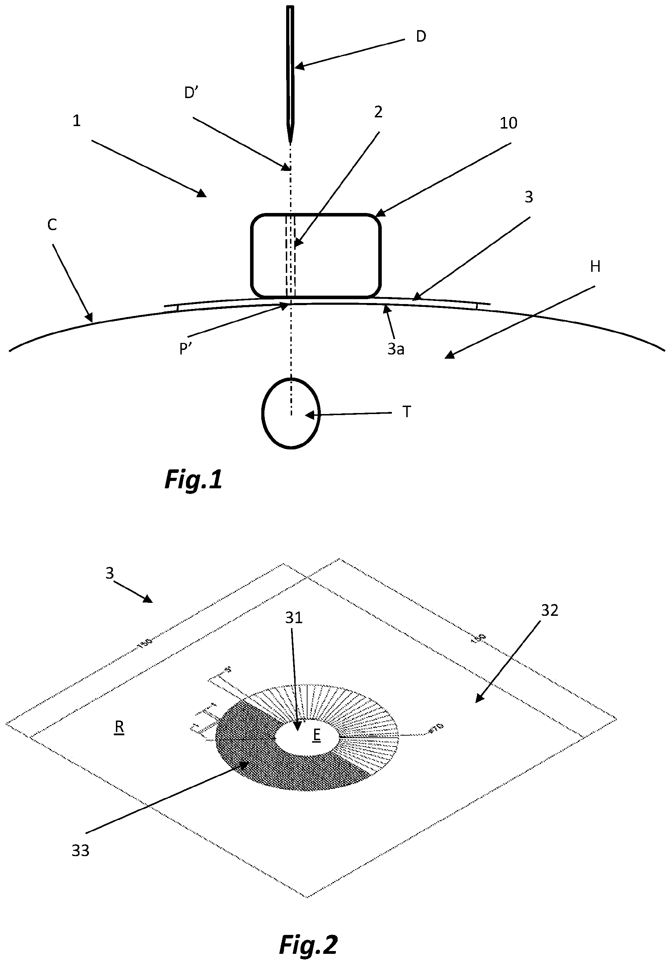

By firstly referring to Figure 1, an operating configuration of a support device, or element, 1 according to the present invention is shown.

Said support element 1 is intended to be applied to a patient's body, in particular to the body surface C, so as to guide the motion of a treatment medical device, or instrument, D during the procedures for inserting (or extracting) the latter in the patient's body.

The invention preferably is applied in combination with treatment instruments D, for example probes or needles, and preferred use destination in the procedures in which such instrument crosses a healthy tissue H to reach a treatment region at a target tissue T to be treated.

The insertion (or extraction) direction, or trajectory, - designated with the reference D' - of the instrument D through the support element 10, intercepts the body surface C in one access point P' of the instrument in the patient's body.

In general terms, the support element 1 comprises a main body 10 carrying at least an opening 2 configured to constrain the motion of the treatment device D when crossing the support element 1 and to determine at least one of said access point P'.

As it will be described more in details hereinafter with reference to the preferred embodiments of the invention, the support element 1 has a main body 10 comprising a first and a second component which can be coupled to each other along a coupling direction. Said coupling direction is orthogonal to a contact surface of the first component with the patient's body.

With further reference to Figure 2, the device 1 of the invention further comprises anchoring means 3 configured for a reversible anchoring of the support element 1 to the patient's body.

In particular, said anchoring means 3 is interposed between the first and the second component and comprises a first face (not visible in figure) having an adhesive surface, or layer 3a intended to adhere to the patient's body. In a preferred embodiment, the adhesive surface 3a is of biocompatible type and comprises a bi-adhesive film.

Moreover, the support element 1 comprises locking means of the first and second component of the main body 10.

Advantageously, such locking means is movable and can assume an operating condition in which the above-mentioned components are relatively locked in such a way as to retain therebetween the anchoring means 3.

Under said operating condition, the locking means prevents the position of the access point P' from varying with respect to the anchoring means 3. In other terms, under said operating conditions the locking means and the anchoring means 3 are integral to each other.

A positioning condition of the locking means instead allows the mobility of the first and second component with respect to the anchoring means 3. Under such condition said components are movable, in particular rotatable and translatable, in an adjustment plane R of the position of the access point P' in the patient's body. Under said positioning condition, the locking means then allows to vary the access point P' in the adjustment plane R.

Said adjustment plane R is parallel to the contact surface 3a with the patient's body, of the main body 10 of the device 1.

It will be then appreciated that, thanks to the specific structural configuration according to the present invention, both a rotation (on itself) and translation motion of the support element 10 with respect to the anchoring means is possible, even under a condition applied to the patient's body.

That is, it is possible to guarantee a modification of the positioning of the access point P' of the treatment instrument 1 in a relative adjustment plane R, subsequently to the anchoring of the support element 1 to the patient's body or even during the introduction (or extraction) procedure itself.

As it is visible in the drawings, said anchoring means 3 comprises a flexible element, for example with planar development, having two opposite faces.

A first advantage of such solution is associated to the possibility of adapting to any geometry of the patient's body. Moreover, if said flexible element is made of non-woven fabric, the anchoring means 3 can be perforated in any point by the treatment instrument D during its insertion in the patient's body.

Preferably, the anchoring means 3 is made of sterilizable material.

Preferably, the anchoring means 3 comprises a through-opening 31 defining the stroke area E available for the motion of the main body 10 in the adjustment plane R.

In the illustrated examples, said through-opening 31 has a circular shape and it is crossed by the main body 10. Different geometries of said through-opening can be provided.

As it can be seen, the anchoring means 3 further comprises a face 32 facing towards the operator, for example a face opposite to the above-mentioned adhesive surface 3a, carrying visual reference means 33 associated to the position of the access point P' with respect to the adjustment plane R.

In a preferred embodiment, said visual reference means 33 surrounds the opening 31 and comprises a Cartesian or radial map to help the operator in measuring the translation or rotation applied to the main body 10 during the adjustment phase. In the example of Figure 2, said map comprises a Cartesian half and a radial half, with the purpose of facilitating the operator in measuring both the translation and the rotation applied to the main body 10 during the adjustment phase.

The particular configuration of the anchoring means 3 then guarantees maximum motion freedom to the main body 10 and, consequently, to adjust the access point P', by simplifying the overall design of the support element 1 which does not require the anchoring means 3 provided with openings to allow the passage of the treatment instrument 1.

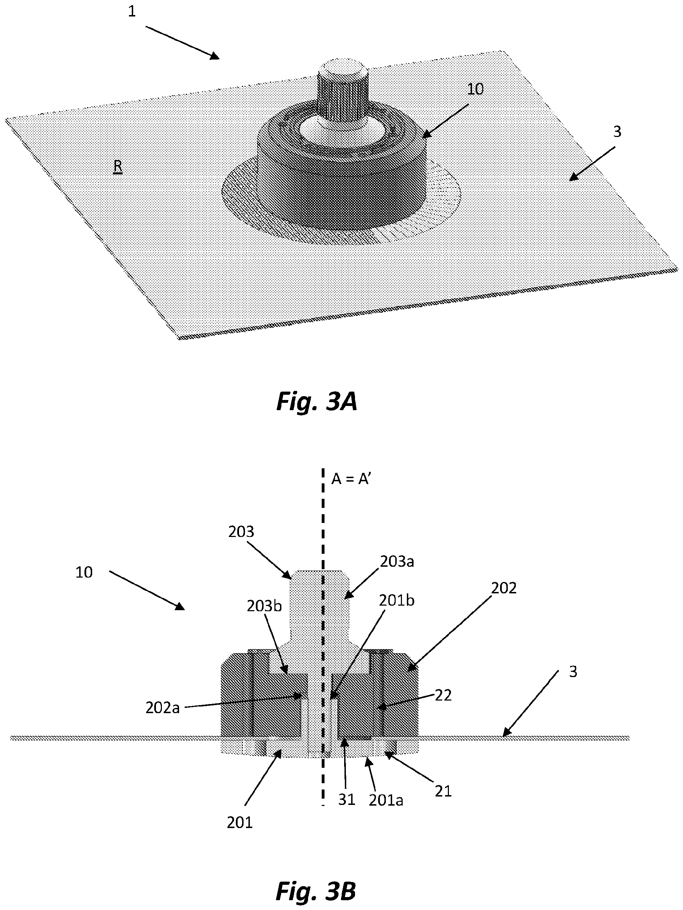

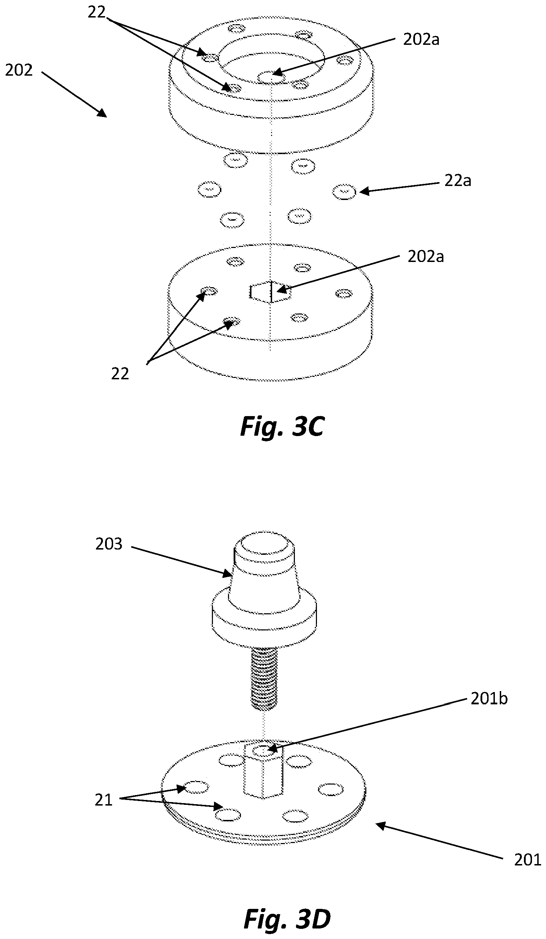

By referring now to Figures 3A-3D the invention according to a first embodiment is illustrated. As it is visible, the support element 10 comprises a first component 201 and a second component 202, coupled to each other, along a coupling direction A orthogonal to the contact surface 201a of the first component 201 with the patient's body.

The locking means 203 is configured to allow a relative locking between said first and second element 201, 202 along a locking direction A' parallel, or coincident, with said coupling direction A.

In the illustrated example, the locking means comprises an engagement element 203 which can be inserted coaxially to a seat 201b obtained in the first component 201.

Said engagement element preferably comprises a gripping portion 203a to facilitate the actuation by the operator.

Said seat 201b and said engagement element 203 preferably comprise mechanical interference means configured to engage mutually and tighten therebetween the first and second component 201, 202. Said mechanical interference means can include a threaded coupling.

The first element 201 comprises a substantially disc-like portion and said seat 201b protrudes vertically from the disc-like portion crossing the through-opening 31 of the anchoring means 3.

The second component 202 comprises a housing 202a apt to receive the seat 201b of the first component 201. For example, said seat 201b and said housing 202a can be shaped with corresponding interlocking profiles, for example with polygonal geometry, so as to prevent a relative rotation of the first and second component 201, 202.

Upon a rotation of the engagement element 203, the two components 201, 202 move approaching along the locking direction A' until the operating condition in which the anchoring means 3, interposed therebetween, are locked. Under locking condition, an abutment surface 203b of the engagement element 203 is in contact with a shoulder of the second component 202.

Upon a reverse rotation of the engagement element 203, the locking of the anchoring means 3 by the first and the second component 201, 202 is released.

As previously mentioned, the components of the main body 10 comprise corresponding through-openings 2 configured to constrain the motion of the treatment device 1 when crossing the support element 10 and to determine at least one access point P' on the patient's body.

With reference to Figure 3B, under coupled condition it can be seen that the through-opening obtained in the first component 201 and designated with reference 21 is aligned with the through-opening obtained in the second component 202 and designated with reference 22.

Thanks to the above-mentioned interlocking profiles, advantageously, said seat 201a and said housing 202a are respectively shaped so that even during the coupling phase of two components, the corresponding through-openings 21, 22 are aligned to each other.

In general terms, the through-openings 2 carried by the support element 10 are preferably circular holes.

The first and second component advantageously comprise a plurality of corresponding through-openings 2 distributed in such a way as to constrain the motion of the treatment device D along directions respectively parallel to each other. Such directions can be orthogonal or tilted with respect to the adjustment plane R.

According to a preferred embodiment, the device 1 of the invention comprises six pairs of corresponding through-openings 2 positioned in such a way as to determine respective access points P, grouped in triplets and arranged at the vertices of corresponding equilateral triangles comprised in said adjustment plane R.

Going back to the example of Figure 3A-3D, the main body 10 comprises six pairs of corresponding through-openings. Such through-openings are designated as whole with reference 21 for the first component 201 and designated as a whole with reference 22 for the second component 202.

Advantageously, the through-openings 22 of the second component 202 can carry retaining means 22a, such as for example O-rings, having a smaller internal diameter than the external diameter of the treatment instrument D intended to be received by each one of said openings 22.

In this way it is possible to generate suitable dynamic friction in the motion of said instrument D so that its free motion is prevented in case it is not kept in position by the operator, for example during the acquisition of TC scanning, but at the same time it can be easily moved during its insertion (or extraction) towards (or from) the target tissue T.

In order to house suitably such retaining means 22a, the second component 202 can be advantageously divided into two coupling portions, by defining suitable grooves to house the retaining means 22a at said through-openings 22.

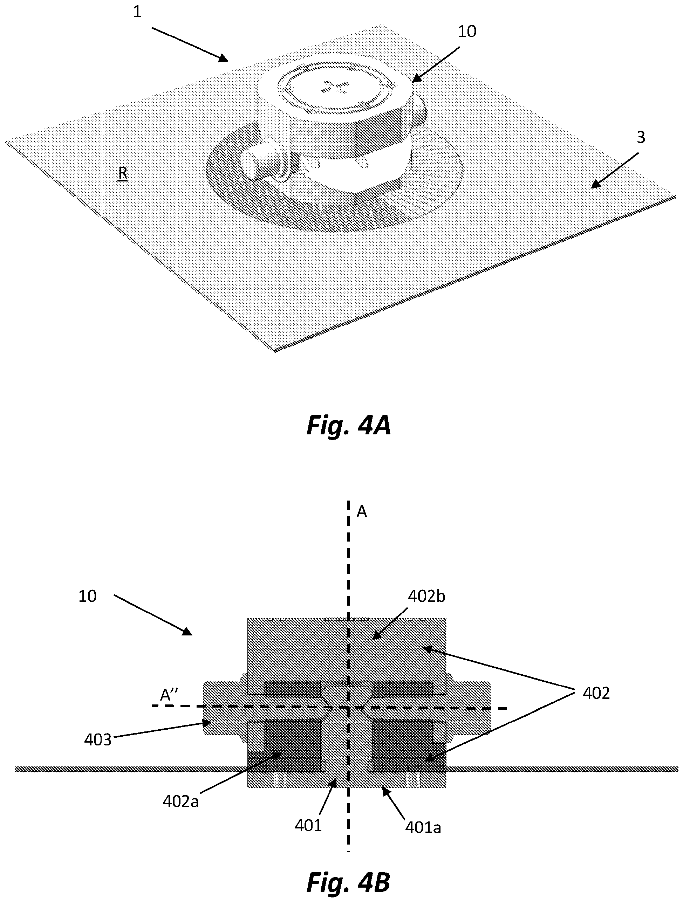

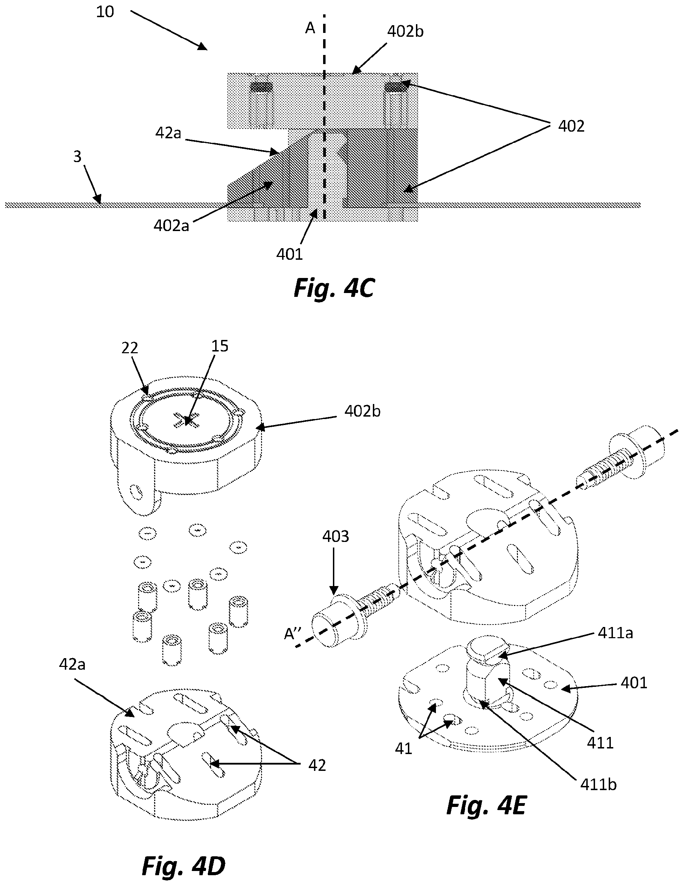

Figures 4A to 4E show the support element 1 of the invention according to a second embodiment which will be described with reference to the features which differentiate it from the embodiment illustrated previously with reference to Figures 3A-3D.

In this case, the locking means 403 is configured to allow a relative locking between the first and second element 401, 402 along a locking direction A" orthogonal to said coupling direction A.

In particular, the locking direction A" represents the direction therealong the locking means 403 is movable to obtain an axial locking of the first and of the second element 401, 402, that is a locking which prevents a motion of said first and second element 401, 402 along said coupling direction A.

Advantageously, the second component 402 comprises a first body 402a and a second body 402b rotatably coupled along said orthogonal locking direction A".

In particular, the first body 402a has a surface 42a coupling with the second body 402b which is comprised in two planes incident to each other. The relative rotation between the first body 402a and the second body 402b takes place around an axis parallel to the locking direction A".

Such shape can allow the rotation of the second body 402b on the first body 402a even under said operating condition in which the first 401 and the second component 402 are locked to each other.

It will be appreciated that such configuration further allows to obtain a tilting of the trajectory D' of the instrument D through the support element 1. In embodiments the through-openings carried by the first component 401 and by the first body 402a can include suitably holes or slots to guarantee the crossing of the support device 1 by the treatment instrument D.

As it can be seen in Figures 4D and 4E, such openings are designated as a whole with reference 41 for the first component 401 and with reference 42 for the first body 402a of the second component 402.

Moreover, the first component 401 comprises a pin element 411 protruding from the disc-like portion, said pin element 411 having a distal end 411a shaped like "V".

The locking means 403, preferably in the form of a threaded engagement element or two threaded engagement elements which can be inserted in opposed way along said locking direction A", interferes mechanically with said distal end 411a by locking the relative motion of the second component 402 with respect to the first component 401.

With the purpose of widening the stroke area E available for the motion of the main body 10 in the adjustment plane R, the pin element 411 can include a throat 411b at the disc-like portion.

Moreover, it is possible to provide for the presence of centering means with the purpose of facilitating the centering of the support element 1 during the procedures in TAC. Said centering means can include for example a cross 15 carved on an external face of the second component 402 and, in case a plurality of corresponding through-openings 22 is provided, centred with respect to the distribution of the latter. Moreover, it is possible to provide for circular breaks on said external face to identify different triplets of equidistant openings 22.

According to an unclaimed aspect of the present invention, then, a method is provided to guide treatment medical devices at a target tissue inside the patient's body.

In general terms, the method provides for providing a support device 1 according to any one of the previously described embodiments and for applying said device 1 on the patient's body through the anchoring means 3.

It is possible to obtain then an access (inlet/outlet) trajectory D' of the treatment medical device D in the patient's body. In particular, at least one access point P' on the body surface C is detected at the through-openings obtained in the main body 10 of the device 1.

Said trajectory D' is compared to a predetermined reference trajectory D*, for example through remote *imaging* techniques such as magnetic resonance and/or computed tomography.

In case the access trajectory D' is different from the reference trajectory D*, one provides for moving the main body 10 on the adjustment plane R to obtain at least a new access point P" different from the previously determined access point P'.

A new access trajectory D" corresponds to point P" and, in case the latter trajectory coincides with the reference trajectory D*, the locking means is activated to lock the support device 1 under the operating condition.

The wished positioning of the support device 1 is then obtained and it is possible to proceed with inserting/extracting the medical device D in the patient's body to reach the target tissue T and to administer the required treatment.

The present invention has been sofar described with reference to preferred embodiments thereof. It is to be meant that each one of the technical solutions implemented in the preferred embodiments, herein described by way of example, could advantageously be combined differently to each other, to create other embodiments, belonging to the same inventive core and however all within the protective scope of the herebelow reported claims.

## Claims

1. A support element (1) configured to guide the insertion and/or the extraction of a treatment medical device (D) in/from a patient's body at a target tissue (T), which support element (1) comprises:
- a main body (10) comprising a first (201; 401) and a second (202; 402) component which can be coupled together along a coupling direction (A), said coupling direction (A) being orthogonal to a contact surface (201a; 401a) of said first component (201; 401) with the patient's body,
- locking means (203; 403) of said first (201; 401) and second (202; 402) component,
- reversible anchoring means (3) of the support element (1) to the patient's body, said anchoring means (3) being interposed between said first (201; 401) and second (202; 402) component,
wherein said first (201; 401) and second (202; 402) component comprise corresponding through-openings (21, 22) configured to constrain a motion of the treatment device (D) passing through the support element (1) and to determine at least one access point (P') on the patient's body,
wherein said locking means (203; 403) are movable between an operating condition, wherein said first (201; 401) and said second (202; 402) component are relatively locked in such a way as to retain integrally therebetween said anchoring means (3), and a positioning condition, wherein said first (201; 401) and second (202; 402) component can be rotated and translated with respect to said anchoring means (3) in an adjustment plane (R) of the position of said at least one access point (P'), said adjustment plane (R) being parallel to said contact surface (201a; 401a),
**characterised in that**
said anchoring means (3) is made of non-woven fabric, comprises a flexible element which develops in said adjustment plane (R) and it is provided with an adhesive surface (3a) intended to adhere to the patient's body wherein said first (201; 401) and second (202; 402) component comprise a plurality of said corresponding through-openings (21, 22) distributed in such a way as to constrain the motion of the treatment device (D) along directions respectively parallel to each other.

2. The support element (1) according to claim 1, wherein said anchoring means (3) comprises visual reference means (33) associated to the position of said at least one access point (P') with respect to said adjustment plane (R).

3. The support element (1) according to any one of the preceding claims, wherein said corresponding through-openings (21, 22) internally carry retaining means (22a) configured to generate friction during a sliding of the treatment device (D) when crossing the support element (1).

4. The support element (1) according to any of the preceding claim, comprising six pairs of corresponding through-openings (21, 22) positioned in such a way as to determine respective access points grouped in triplets and arranged at the vertices of corresponding equilateral triangles comprised in said adjustment plane (R).

5. The support element (1) according to any one of the preceding claims, wherein said locking means (203) is configured to allow a relative locking between said first (201) and second (202) component along a locking direction (A') parallel to said coupling direction (A).

6. The support element (1) according to any of the claims 1 to 4, wherein said locking means (403) is configured to allow a relative locking between said first (401) and second (402) component along a locking direction (A") orthogonal to said coupling direction (A).

7. The support element (1) according to the preceding claim, wherein said second component (402) comprises a first (402a) and a second (402b) body rotatably coupled to each other.

8. The kit comprising a support element (1) according to any one of the preceding claims and a treatment medical device (D), in particular a probe or a needle.

## Patentansprüche

1. Unterstützungselement (1), das konfiguriert ist, um das Einsetzen und/oder die Extraktion einer medizinischen Behandlungsvorrichtung (D) in einen/von einem Körper eines Patienten an einem Zielgewebe (T) zu führen, wobei das Unterstützungselement (1) umfasst:
- einen Hauptkörper (10), umfassend eine erste (201; 401) und eine zweite (202; 402) Komponente, die entlang einer Kopplungsrichtung (A) miteinander koppelbar ist, wobei die Kopplungsrichtung (A) orthogonal zu einer Kontaktoberfläche (201a; 401a) der ersten Komponente (201; 401) mit dem Körper des Patienten ist,
- Verriegelungsmittel (203; 403) der ersten (201; 401) und der zweiten (202; 402) Komponente,
- ein reversibles Verankerungsmittel (3) des Unterstützungselements (1) an dem Körper des Patienten, wobei das Verankerungsmittel (3) zwischen der ersten (201; 401) und der zweiten (202; 402) Komponente angeordnet ist,
wobei die erste (201; 401) und die zweite (202; 402) Komponente entsprechende Durchgangsöffnungen (21, 22) umfassen, die konfiguriert sind, um eine Bewegung der Behandlungsvorrichtung (D), die durch das Trägerelement (1) verläuft, zu begrenzen und mindestens einen Zugangspunkt (P') auf dem Körper des Patienten zu bestimmen,
wobei die Verriegelungsmittel (203; 403) zwischen einem Betriebszustand, wobei die erste (201; 401) und die zweite (202; 402) Komponente derart relativ verriegelt sind, um dazwischen das Verankerungsmittel (3) integral zu halten, und einem Positionierungszustand bewegbar sind, wobei die erste (201; 401) und die zweite (202; 402) Komponente in Bezug auf das Verankerungsmittel (3) in einer Einstellebene (R) der Position des mindestens einen Zugangspunkts (P') gedreht und verschoben werden können, wobei die Einstellebene (R) parallel zu der Kontaktoberfläche (201a; 401a) ist,
**dadurch gekennzeichnet, dass** das Verankerungsmittel (3) aus Vliesstoff hergestellt ist, ein flexibles Element umfasst, das sich in der Einstellebene (R) entwickelt und mit einer Haftoberfläche (3a) versehen ist, die vorgesehen ist, um an dem Körper des Patienten zu haften,
wobei die erste (201; 401) und die zweite (202; 402) Komponente eine Vielzahl der entsprechenden Durchgangsöffnungen (21, 22) umfassen, die derart verteilt ist, dass sie die Bewegung der Behandlungseinrichtung (D) entlang von Richtungen, jeweils parallel zueinander, begrenzt.

2. Unterstützungselement (1) nach Anspruch 1, wobei das Verankerungsmittel (3) visuelle Referenzmittel (33) umfasst, die der Position des mindestens einen Zugangspunkts (P') in Bezug auf die Einstellebene (R) zugeordnet sind.

3. Unterstützungselement (1) nach einem der vorstehenden Ansprüche, wobei die entsprechenden Durchgangsöffnungen (21, 22) Haltemittel (22a) intern tragen, die konfiguriert sind, um während eines Gleitens der Behandlungseinrichtung (D) bei einem Überqueren des Unterstützungselements (1) Reibung zu erzeugen.

4. Unterstützungselement (1) nach einem der vorstehenden Ansprüche, umfassend sechs Paare von entsprechenden Durchgangsöffnungen (21, 22), die derart positioniert sind, dass jeweilige Zugangspunkte in Tripletten gruppiert und an den Scheitelpunkten entsprechender gleichseitiger Dreiecke angeordnet sind, die in der Einstellebene (R) enthalten sind.

5. Unterstützungselement (1) nach einem der vorstehenden Ansprüche, wobei das Verriegelungsmittel (203) konfiguriert ist, um eine relative Verriegelung zwischen der ersten (201) und der zweiten (202) Komponente entlang einer Verriegelungsrichtung (A') parallel zu der Kopplungsrichtung (A) zu ermöglichen.

6. Unterstützungselement (1) nach einem der Ansprüche 1 bis 4, wobei das Verriegelungsmittel (403) konfiguriert ist, um eine relative Verriegelung zwischen der ersten (401) und der zweiten (402) Komponente entlang einer Verriegelungsrichtung (A") orthogonal zu der Kopplungsrichtung (A) zu ermöglichen.

7. Unterstützungselement (1) nach dem vorstehenden Anspruch, wobei die zweite Komponente (402) einen ersten (402a) und einen zweiten (402b) Körper umfasst, die miteinander drehbar gekoppelt sind.

8. Kit, umfassend ein Unterstützungselement (1) nach einem der vorstehenden Ansprüche und eine medizinische Behandlungsvorrichtung (D), insbesondere eine Sonde oder eine Nadel.

## Revendications

1. Élément de support (1) conçu pour guider l'insertion et/ou l'extraction d'un dispositif médical de traitement (D) dans/depuis le corps d'un patient au niveau d'un tissu cible (T), lequel élément de support (1) comprend :
- un corps principal (10) comprenant un premier (201 ; 401) et un second (202 ; 402) composant qui peuvent être accouplés ensemble le long d'une direction d'accouplement (A), ladite direction d'accouplement (A) étant orthogonale à une surface de contact (201a ; 401a) dudit premier composant (201 ; 401) avec le corps du patient,
- un moyen de verrouillage (203 ; 403) dudit premier (201 ; 401) et dudit second (202 ; 402) composant,
- un moyen d'ancrage (3) réversible de l'élément de support (1) au corps du patient, ledit moyen d'ancrage (3) étant interposé entre ledit premier (201 ; 401) et ledit second (202 ; 402) composant,
dans lequel ledit premier (201 ; 401) et ledit second (202 ; 402) composant comprennent des ouvertures traversantes correspondantes (21, 22) conçues pour contraindre un mouvement du dispositif de traitement (D) passant à travers l'élément de support (1) et pour déterminer au moins un point d'accès (P') sur le corps du patient,
dans lequel ledit moyen de verrouillage (203 ; 403) est mobile entre une condition de fonctionnement, dans laquelle ledit premier (201 ; 401) et ledit second (202 ; 402) composant sont verrouillés relativement de manière à retenir d'un seul tenant entre eux ledit moyen d'ancrage (3) et une condition de positionnement, dans laquelle ledit premier (201 ; 401) et ledit second (202 ; 402) composant peuvent être tournés et déplacés en translation par rapport audit moyen d'ancrage (3) dans un plan de réglage (R) de la position dudit au moins un point d'accès (P'), ledit plan de réglage (R) étant parallèle à ladite surface de contact (201a ; 401a),
**caractérisé en ce que** ledit moyen d'ancrage (3) est constitué de textile non tissé, comprend un élément souple qui se développe dans ledit plan de réglage (R) et il est pourvu d'une surface adhésive (3a) destinée à adhérer au corps du patient
dans lequel ledit premier (201 ; 401) et ledit second (202 ; 402) composant comprennent une pluralité desdites ouvertures traversantes correspondantes (21, 22) réparties de manière à contraindre le mouvement du dispositif de traitement (D) le long des directions respectivement parallèles l'une à l'autre.

2. Élément de support (1) selon la revendication 1, dans lequel ledit moyen d'ancrage (3) comprend un moyen de référence visuel (33) associé à la position dudit au moins un point d'accès (P') par rapport audit plan de réglage (R).

3. Élément de support (1) selon l'une quelconque des revendications précédentes, dans lequel lesdites ouvertures traversantes correspondantes (21, 22) portent intérieurement un moyen de retenue (22a) conçu pour générer un frottement pendant un coulissement du dispositif de traitement (D) lorsqu'il traverse l'élément de support (1).

4. Élément de support (1) selon l'une quelconque revendication précédente, comprenant six paires d'ouvertures traversantes correspondantes (21, 22) positionnées de manière à déterminer des points d'accès respectifs groupés en triplets et agencés au niveau des sommets de triangles équilatéraux correspondants compris dans ledit plan de réglage (R).

5. Élément de support (1) selon l'une quelconque des revendications précédentes, dans lequel ledit moyen de verrouillage (203) est conçu pour permettre un verrouillage relatif entre ledit premier (201) et ledit second (202) composant le long d'une direction de verrouillage (A') parallèle à ladite direction d'accouplement (A).

6. Élément de support (1) selon l'une quelconque des revendications 1 à 4, dans lequel ledit moyen de verrouillage (403) est conçu pour permettre un verrouillage relatif entre ledit premier (401) et ledit second (402) composant le long d'une direction de verrouillage (A") orthogonale à ladite direction d'accouplement (A).

7. Élément de support (1) selon la revendication précédente, dans lequel ledit second composant (402) comprend un premier (402a) et un second corps (402b) accouplés de manière rotative l'un à l'autre.

8. Kit comprenant un élément de support (1) selon l'une quelconque des revendications précédentes et un dispositif médical de traitement (D), en particulier une sonde ou une aiguille.
